# FASCICULE DE BREVET EUROPEEN

(11) **EP 0 687 306 B1**
(45) Date de publication et mention de la délivrance du brevet: **06.12.2000**
(21) Numéro de dépôt: 94906098.2
(22) Date de dépôt: 18.02.1994
(51) Int. Cl.: C12Q 1/68

(54) **SONDES D'ACIDES NUCLEIQUES SPECIFIQUES AU SPIROCHETE BORRELIA BURGDORFERI**
NUKLEINSÄURESONDE SPEZIFISCH FÜR DEN SPIROCHAETEN BORRELIA BURGDORFERI
BORRELIA BURGDORFERI SPIROCHAETE-SPECIFIC NUCLEIC ACID PROBES

(30) Priorité: 19.02.1993 BE 9300161
(43) Date de publication de la demande: 20.12.1995
(73) Titulaire: LA REGION WALLONNE, 1040 Bruxelles (BE)
(72) Inventeur: GODFROID, Edmond, B-1080 Bruxelles (BE); BOLLEN, Alex, B-1701 Itterbeek (BE)
(74) Mandataire: de Kemmeter, François
(86) Numéro de dépôt international: BE9400012
(87) Numéro de publication internationale: WO9419488

(56) Documents cités:
- WO-A-90/04411
- WO-A-92/09703
- WO-A-93/00448
- WO-A-93/04175
- WO-A-93/08306
- JOURNAL OF CLINICAL MICROBIOLOGY vol. 31, no. 2 , Février 1993 , WASHINGTON US pages 340 - 350 WILSKE ET AL.
- GENE. vol. 120 , 1992 , AMSTERDAM NL pages 127 - 128 FELLINGER ET AL.

## Description

La présente invention concerne un agent oligonucléotidique utilisable pour la mise en évidence d'au moins une souche de Borrelia burgdorferi ainsi que les diverses applications dudit agent oligonucléotidique.

Le spirochète Borrelia burgdorferi (Bb) est l'agent causal de la maladie de Lyme. L'hôte vecteur de ce spirochète est un acarien du genre Ixodes, des espèces ricinus en Europe ou dammini et pacificus en Amérique du Nord. Le diagnostic clinique de la maladie repose sur différents signes anatomo-pathologiques (érythème chronique migrant, fièvre, arthrite, méningite et dans les cas les plus graves, myocaydite) [1]. La recherche d'anticorps anti-OspA (glycoprotéine située à la surface de Bb) et/ou anti-flagelline (protéine du flagelle) constitue la méthode principale du diagnostic immuno-biochimique de cette maladie [2]. Cette recherche d'anticorps anti-Borrelia possède néanmoins un certain nombre d'inconvénients. Elle est dépendante de la réactivité du système immunitaire de l'hôte vis-à-vis de Bb, déterminant la sensibilité de ce type de système de détection. Cette méthode immuno-biochimique possède également comme inconvénient de ne pas pouvoir dissocier une éventuelle réaction croisée avec des agents infectieux proches à Bb (par exemple, Treponema pallidum responsable de la syphilis) d'une reconnaissance spécifique [3). Il est donc intéressant de développer de nouveaux outils diagnostiques qui contournent ces problèmes. La détection de l'ADN de Bb selon les méthodes d'hybridation moléculaire qui mettent en jeu une sonde nucléotidique et un ADN cible remplit ces conditions. Ces expériences d'hybridation sont généralement menées sur un support solide (filtre de nitrocellulose ou de nylon) où l'ADN cible est fixé, ou encore, en milieu liquide, par exemple, lors d'une réaction d'amplification génétique connue sous le nom de "Polymerase Chain Reaction" ou PCR nécessitant la présence de l'ADN cible, de deux sondes ou amorces nucléotidiques spécifiques à l'ADN cible et d'une enzyme de polymérisation appelée Taq polymérise (4]. Ces méthodes d'hybridation moléculaire sont d'une grande sensibilité (possibilité de détecter un seul génome bactérien et d'une grande spécificité. Elles ont l'avantage de ne pas dépendre d'une quelconque réaction physiologique ou pathologique de l'organisme hôte infecté.

Les études d'hybridation moléculaire sur génome entier de Bb ont permis de séparer an trois grands groupes, un grand nombre de souches connues [6]. Cette répartition en différents groupes est également mise an évidence an comparent la séquence du gène codant pour la glycoprotéine de surface OspA de différentes souches de Bb par un programme informatique adapté. Les résultats de cette analyse informatique sont corroborés par une étude phylogénétique menée sur le gène codant pour l'ARN ribosomal 16S [5]. L'analyse de cette classification des souches de Bb en relation aux signes cliniques observés au cours de la maladie suggère un lien intéressant entre l'appartenance d'une souche à un groupe déterminé et la pathologie qui y est associée; par exemple, les souches reprises dans le groupe I montrent une propension à provoquer des signes arthritiques tandis que celles appartenant au groupe II provoquent principalement des neuroborélioses [7].

La détection d'une infection par Bb se fait actuellement par la reconnaissance, par le sérum du patent potentiellement infecté, de l'antigène flagellaire purifié, fixé sur plaque de type Elisa. Cette reconnaissance est mise en évidence par l'addition d'un anticorps anti-humain associé à une enzyme de révélation ou une molécule mettant en oeuvre ce type d'enzyme [8].

WO-A-9209703 divulgue de nouveaux réactifs utilisables dans des réactions d'amplification génétique (PCR) pour détecter les séquences d'acides nucléiques de Borrelia dans des tiques suspects et dans d'autres échantillons. Ces réactifs consistent en amorces telles que Osp-A2 et Osp-A4 et sondes oligonucléotidiques p.ex. Osp-A3 codant l'Osp-A (outer surface protein) et d'autres protéines. Les séquences revendiquées OspA2, OspA3 et OspA4 sont toutefois totalement inappropriées à la discrimination entre espèces de Borrelia burgdorferi. De plus, Osp-A3 étant prévu comme sonde et Osp-A4 comme amorce, l'intérêt d'Osp-A4 ou de ses modifications éventuelles en tant que sonde plutôt que comme amorce PCR n'apparaît pas à la lecture du document.

Dans le cadre des études épidémiologiques, du diagnostic, en routine des infections par les différents groupes connus de Bb et du suivi de l'efficacité et de la composition des vaccins protecteurs des infection par Bb, il est utile de développer des méthodes simples, spécifiques et sensibles basées sur l'emploi soit de sondes à ADN synthétique marquées de façon non isotopique utilisables dans des réactions d'hybridation moléculaire sur support solide, soit d'amorces d'ADN synthétique utilisables dans des réactions d'amplification génétique (PCR). A cet effet nous avons identifié par traitement informatique, au sein de la séquence du gène codant pour la glycoprotéine de surface OspA de Bb, des régions susceptibles d'être utilisées comme sondes ou amorces dans des réactions d'hybridation moléculaire.

Un but de la présente invention est de proposer des séquences communes à tous les groupes de souches de Bb, utilisables soit comme amorces au cours de réactions d'amplification génétique (PCR), soit comme sonde de capture dans un test d'hybridation sur support solide où l'ADN cible est pris en "sandwich" entre cette sonde de capture et une sonde de révélation, soit comme sondes de révélation dans ce test d'hybridation sur support solide.

Un autre but de la présente invention est de proposer des séquences spécifiques de chacun des groupes de souches de Bb, utilisables comme amorces au cours de réactions d'amplification génétique (PCR) ou comme sondes de révélation ou de capture dans le test d'hybridation sur support solide évoqué ci-dessus.

Ces différents buts sont atteints au moyen d'un agent oligonucléotidique tel que défini dans la revendication principale 1 et tel que précité dans les revendications subordonnées 2 à 12.

Parmi les sequences qui y sont définies, on distingue les séquences communes à tous les groupes de souches de Borrelia burgdorferi et les séquences spécifiques de groupes.

L'invention vise également un procédé de détection de Borrelia burgdorferi tel que défini dans les revendications principales 13 et 14 et tel que précité dans les revendications subordonnées 15 à 25.

L'utilisation d'un ou plusieurs oligonucléotides selon l'invention, telle que défini à la revendication 26, fait également partie de la présente invention.

### 1. Séquence communes à tous les groupes de souches de Borrelia burgdorferi.

### A. Utilisables au cours de réactions d'amplification génétique (PCR).

La présente invention a donc pour objet des amorces d'acides nucléiques utilisables pour l'amplification génétique de l'ADN de tous les groupes de souches connues de Bb. La comparaison de la séquence du gène codant pour la glycoprotéine de surface OspA, par un programme informatique adapté, permet de cibler ce type d'amorces.

La réaction PCR nécessite l'utilisation de 2 amorces d'acides nucléiques. L'une est située sur le brin codant; l'autre se situe sur le brin complémentaire en aval de la première amorce. Le tableau 1, ci-dessous, présente la séquence de 2 paires d'amorces permettant l'amplification d'un fragment d'ADN au sein de la séquence du gène OspA de tous les groupes de souches Bb. Ce tableau présente également la position de ces amorces sur la séquence connue du gène OspA de la souche B31, leur température de demi-hybridation et leur taille respective.

A, T, C, G représentent les nucléotides correspondant aux bases adénine, thymine, cytosine et guanine, respectivement.

**Tableau 1**

| Sondes | Séquences | Long. | Tm | Position |
|---|---|---|---|---|
| OspApc1 | AATAGGTCTA ATATTAGCCT TAATAGC | 27 | 70°C | 21 - 47 |
| OspApc2 | CTAGTGTTTT GCCATCTTCT TTGAAAA | 27 | 70°C | 328 - 302 |

Les séquences dans ce tableau sont présentées dans l'orientation 5'→3'. La séquence de l'amorce OspApc2 est présentée en mode complémentaire inverse à la séquence du brin codant (ou de la fibre directe).

L'utilisation des amorces OspApc1/OspApc2 dans une réaction PCR génère un fragment d'ADN de 282 paires de bases (pb).
pc signifie "primer conservé".

### B. Utilisables pour la capture de l'ADN issu de tous les groupes de souches de Borrelia burgdorferi.

La présente invention a également pour objet une séquence commune à tous les groupes de souches de Bb utilisables pour la "capture" de l'ADN issu de tous ces groupes dans un test, dit de sandwich, où des sondes oligonucléotidiques spécifiques à chacun des groupes ou communes à tous les groupes de souches de Bb servent à la révélation de l'hybride moléculaire sonde de "capture" - ADN de Bb. Une telle sonde de "capture" peut être fixée par un lien covalent à un support adéquat. Dans un tel système, l'ADN cible est pris en sandwich entre l'oligonucléotide de capture et la sonde de révélation.

La séquence de cet oligonucléotide de capture commun à tous les groupes de souches de Bb a pu être définie par comparaison informatique des séquences du gène codant pour la glycoprotéine de surface OspA. La séquence de cet oligonucléotide est la suivante:
Bb50: 5'-ATGAAAAAAT ATTTATTGGG AATAGGTCTA ATATTAGCCT TAATAGCATG-3'

La séquence de cet oligomère comprend 50 nucléotides: elle se situe sur le brin codant du gène OspA et s'étend de la position 1 à 50 de la séquence codante du gène OspA de la souche B31.

### C. Utilisables pour la révélation d'une réaction d'hybridation entre la sonde de capture et l'ADN cible.

La présente invention a également pour objet des sondes utilisables pour l'identification de l'espèce Bb par rapport aux autres bactéries. Ces sondes oligonucléotidiques communes à tous les groupes de souches de Bb permettent de révéler dans un test d'hybridation, tel qu'il est décrit ci-dessus au point 1.B, la présence d'un hybride moléculaire constitué par la sonde de capture et l'ADN cible.

Ces sondes de révélation sont modifiées chimiquement par l'addition en 5' et/ou en 3' d'un ou plusieurs groupement(s) moléculaire(s) ayant une affinité pour une molécule associée à une enzyme, par exemple, la phosphatase alcaline. Cette enzyme peut réagir soit avec un substrat chromogénique incolore et soluble donnant un produit de réaction coloré ou un substrat chémiluminescent, par exemple l'AMPPD™ ou le CSPD™ (Tropix, USA).

Ces sondes de révélations comprennent une séquence d'acides nucléiques qui est la suivante:
- pour l'oligomère Bb35bio:
   5'-GCAACAGTAG ACAAGCTTGA GCTTAAAGGA ACTTC-3'
- pour l'oligomère Bb23bio:
   5'-TTTTCAAAGA AGATGGCAAA ACA-3'

Les oligomères Bb35bio et Bb23bio, communs à tous les groupes de souches de Bb comprennent respectivement 35 et 23 monomères. Leur séquence s'étend respectivement de la position 168 à 200 et de la position 302 à 324 de la séquence codante du gène OspA de la souche B31.

### 2. Séquences spécifiques de groupes.

### A. Utilisables au cours de réaction d'amplification génétique (PCR).

La présente invention a aussi pour objet des amorces spécifiques de chacun des groupes de souches de Bb utilisables dans une réaction PCR. Les amorces de type I, notées BbGI1 et BbGI2, permettent l'amplification d'un fragment d'ADN caractéristique des souches de ce groupe. Les amorces des groupes II, symbolisées par BbGII1 et BbGII2, permettent l'amplification d'un fragment d'ADN caractéristique du groupe II tandis que les amorces du groupe III, symbolisées par BbGIII1, BbGIII2 et BbGIII3, permettent lorsqu'elles sont combinées par paire, par exemple BbGIII1 et BbGIII2, l'amplification d'un fragment d'ADN caractéristique des souches du groupe III.

La séquence de ces amorces spécifiques de groupes, présentée de l'extrémité 5' vers l'extrémité 3', est la suivante:
- pour les souches du groupe I, les amorces:
   BbGI1: 5'-AACAAAGACG GCAAGTACGA TCTAATT-3'
   BbGI2: 5'-TTACAGTAAT TGTTAAAGTT GAAGTGCC-3'
- pour les souches du groupe II, les amorces:
   BbGII1: 5'-TGATAAAAAC AACGGTTCTG GAAC-3'
   BbGII2: 5'-GTAACTTTCA ATGTTGTTTT GCCG-3'
- pour les souches du groupe III, les amorces:
   BbGIII1: 5'-GAAAAAGGTG AATTGTCTGC AAAAACC-3'
   BbGIII2: 5'-TTCCAATGTT ACTTTATCAT TAGCTACTT-3'
   BbGIII3: 5'-TAAAGACAAA ACATCAACAG ATGAAATG-3'

Les oligomères BbGI1 et BbGI2 débutent respectivement à la position 139 et 682 sur la séquence codante du gène OspA de la souche B31 (souche du groupe I). Ils sont constitués respectivement de 27 et 28 nucléotides. Leur température de demi-hybridation est de 74°C. L'oligomère BbGI2 est présenté en mode complémentaire inverse au brin codant. L'utilisation de ces deux oligomères dans une réaction PCR permet l'amplification d'un fragment d'ADN de 544 paires de bases (pb).

Les oligomères BbGII1 et BbGII2 débutent respectivement à la position 201 et 545 sur la séquence codante du gène OspA de la souche B29 (souche du groupe II). Ils sont constitués tous deux de 24 nucléotides. Leur température de demi-hybridation est de 66°C. L'oligomère BbGII2 est présenté en mode complémentaire inverse au brin codant. L'utilisation de ces deux oligomères dans une réaction PCR permet l'amplification d'un fragment d'ADN de 345 paires de bases (pb).

Les oligomères BbGIII1, BbGIII2 et BbGIII3 débutent respectivement à la position 381, 536 et 347 sur la séquence codante du gène OspA de la souche AcaI (souche du groupe III). Ils sont constitués respectivement de 27, 29 et 28 nucléotides. La température de demi-hybridation est de 74°C pour les oligomères BbGIII1 et BbGIII2, et de 72°C pour l'oligomère BbGIII3. L'oligomère BbGIII2 est présenté en mode complémentaire inverse au brin codant. L'utilisation des oligomères BbGIII1 et BbGIII2 dans une réaction PCR permet l'amplification d'un fragment d'ADN de 155 paires de bases (pb). La paire d'oligomères BbGIII3/BbGIII2 génère après amplification un fragment d'ADN de 188 pb.

### B. Utilisables pour la détection directe ou indirecte, lors d'une réaction d'hybridation ADN/ADN, des différents groupes de souches de Borrelia burgdorferi.

La présente invention a aussi pour objet des sondes d'acides nucléiques spécifiques à chacun des groupes de souches de Bb utilisables pour la détection directe ou indirecte des différents groupes de souches de Bb. La détection directe peut se faire par hybridation de l'ADN cible fixé sur un support solide, par exemple: la nitrocellulose ou le nylon, par une de ces sondes appartenant à un des groupes connus de souche de Bb. La détection indirecte peut se faire via le système d'hybridation moléculaire "sandwich" exposé au point 1.B. Dans ce cas, la sonde de capture peut être soit une sonde commune à tous les groupes de souches de Bb soit une sonde spécifique à chacun des groupes. De telles sondes de "capture" peuvent être également fixées par un lien covalent à un support adéquat. Dans ce test de "sandwich", les oligonucléotides de révélation seront soit l'oligomère Bb35bio ou Bb23bio lorsque la sonde de capture est spécifique à chacun des groupes de Bb, soit l'oligomère BbGI23, BbGIB23 ou BbGIII28 lorsque la sonde de capture est commune à tous les groupes de Bb. Ces sondes sont associées à une molécule ayant une affinité pour une molécule associée à une enzyme, par exemple, la phosphatase alcaline. De même, dans un système de détection directe, les sondes sont modifiées comme décrit ci-dessus.

La séquence présentée dans le sens 5'-3', de ces oligomères est la suivante:
- pour le groupe I, l'oligomère BbGI23:
   5'-CTGCAGCTTG GAATTCAGGC ACT-3'
- pour le groupe II, l'oligomère BbGII23:
   5'-ACTCTAGCTG CTGACGGCAA AAC-3
- pour le groupe III, l'oligomère BbGIII28:
   5'-AGGAAAAGTA GCTAATGATA AAGTAACA-3'

L'oligomère BbGI23 commence à la position 638 pour se terminer à la position 660 de la séquence codante du gène OspA de la souche B31 (souche du groupe I). Il est constitué de 23 nucléotides. Sa température de demi-hybridation est de 70°C.

Loligomère BbGII23 commence à la position 508 pour se terminer à la position 530 de la séquence codante du gène OspA de la souche B29 (souche du groupe II). Il est constitué de 23 nucléotides. Sa température de demi-hybridation est de 70°C.

L'oligomère BbGIII28 commence à la position 503 pour se terminer à la position 530 de la séquence codante du gène OspA de la souche AcaI (souche du groupe III). Il est constitué de 28 nucléotides. Sa température de demi-hybridation est de 70°C.

Ci-après on donne quelques exemples d'applications des agents oligonucléotidiques décrits.

### A. Diagnostic de Borrelia burgdorferi (Bb) dans les liquides biologiques des sujets infectés.

Le spirochète Bb infecte l'animal hôte suite à une morsure par une tique (Ixodes ricinus, pour l'Europe) infectée. A l'endroit de la morsure, il se développe rapidement (quelques heures) un érythème annulaire et puis un érythème chronique migrant. Le spirochète s'installe ensuite dans différents organes et tissus de l'hôte (vessie, système nerveux central, tissus cartilagineux) après avoir transité par le sang et le foie. Il est donc intéressant d'analyser par un système de détection approprié (PCR avec amorces originales spécifiques à Bb ou test d'hybridation en "sandwich" avec sondes nucléotidiques originales spécifiques à Bb) les liquides biologiques qui peuvent être potentiellement infectés par Bb suite à une morsure par une tique. Le sang et les urines constituent des liquides de choix vu la facilité avec laquelle ils sont prélevés. Lorsque des signes articulaires (arthrite) ou neurologiques apparaissent, il est intéressant d'utiliser ces systèmes originaux d'investigation moléculaire sur les liquides synovial et céphalo-rachidien.

### B. Applications humaine et vétérinaire.

L'utilisation de notre système de détection, basée sur des sondes et/ou amorces moléculaires spécifiques à Bb, à des échantillons humains est démontrée au point 3.A., développé ci-dessus. Ce système de détection peut être étendu à des échantillons d'origine animale. Les chats, les chiens, les bovidés et les équidés peuvent être des animaux hôtes de Bb. La boiterie du cheval peut trouver son origine par l'infection de l'articulation tibio-tarsienne par Bb. Certains auteurs ont montré la présence de Bb dans le lait de vache. Il est donc intéressant d'utiliser le système de détection que nous proposons sur des échantillons d'origine vétérinaire qui seraient potentiellement infectés par Bb.

### C. Aide à la thérapeutique de l'infection.

Les études d'hybridation moléculaire entre génome entier de Bb ont permis de séparer un grand nombre de souches connues en 3 grands groupes. La comparaison informatique de la séquence connue du gène OspA de plusieurs souches de Bb a également mis en évidence la ségrégation de ces différentes souches de Bb en 3 grands groupes. L'analyse de cette classification des souches de Bb en relation aux signes cliniques observés au cours de la maladie montre un lien intéressant entre l'appartenance d'une souche à un groupe déterminé et la pathologie qui y est associée; par exemple, les souches reprises dans le groupe I montrent une propension à provoquer des signes arthritiques tandis que celles appartenant au groupe II provoquent principalement des neuroborélioses. Les souches du groupe III, quant à elles, provoquent essentiellement des acrodermatites.

La relation entre l'appartenance à un groupe et une pathologie spécifique développée au cours de stades plus avancés de la maladie est une notion importante pour le clinicien. Celui-ci adaptera sa médication (type d'antibiotique, posologie, durée du traitement) en fonction de différents résultats qu'il aura engrangé lors de l'analyse des symptômes et de l'étude biochimique des différents liquides biologiques prélevés. La détection rapide, spécifique et fiable du groupe de Bb infectant le patient à des stades précoces de la maladie donnera une indication précieuse au clinicien quant au type d'évolution auquel il peut s'attendre si la médication n'a pas les effets désirés. Cette réponse diagnostique permettra au clinicien d'adapter son traitement afin d'éviter une évolution de la maladie vers des stades plus préoccupants du point de vue clinique (atteinte neurologique et cardiaque, chronicité de la maladie). L'utilisation de notre système de détection, basée sur des sondes et/ou amorces moléculaires originales, spécifiques à chacun des groupes de souches de Bb, permet de rencontrer cet objectif.

### D. Epidémiologie et étude des animaux réservoirs.

Une étude épidémiologique de la maladie de Lyme comporte deux aspects: l'un, comprend une étude du vecteur de la maladie, Ixodes ricinus; l'autre, comprend l'analyse des animaux réservoirs (sauvages et domestiques). Le vecteur de cette maladie est un acarien (Ixodes ricinus) qui se développe dans des sous-bois à haute teneur en humidité en trois stades: larve, nymphe et adulte. A chaque phase de développement, Ixodes ricinus réalise un repas sanguin au cours duquel il peut s'infecter s'il est en contact avec un animal contaminé par Bb. L'animal sauvage hôte, par excellence, de Bb est le chevreuil en raison de la superposition du biotope du chevreuil à celui de la tique. Néanmoins, les animaux sauvages hôtes, réservoirs potentiels de la maladie, sont très divers. Ils peuvent être des rongeurs (rat des champs, lièvre, lapin,...), des oiseaux (merle, moineau,...), des suidés (sanglier), des cervidés (chevreuil, daim, cerf) ou encore des canidés (loup, renard).

Une étude épidémiologique du vecteur de Bb et des animaux sauvages cités ci-dessus permettra non seulement de définir les zones géographiques à haut risque d'infection, mais aussi de mesurer le degré d'infection des trois stades de développement du vecteur et des animaux sauvages, réservoirs potentiels du spirochète. La méthode de détection, telle que nous la proposons, basée sur l'hybridation moléculaire entre l'ADN cible et une sonde ou une paire d'amorces originale, spécifique à Bb permet d'assurer ce type d'objectif.

### E. Suivi de l'efficacité de la vaccination.

La vaccination de patients vivant dans des régions géographiques à haute endémicité de Bb ou de patients qui par leur activité (garde-chasse, garde-forestier, agriculteur, promeneur) côtoient ces régions constitue la méthode prophylactique permettant d'éviter les signes cliniques douloureux et invalidants associés à la maladie de Lyme. Néanmoins, un certain nombre de patients peuvent échapper à la protection ciblée par le vaccin. Il est donc intéressant de suivre l'efficacité des vaccins proposés par la détection de Bb chez les patients vaccinés présentant des signes cliniques associés à la maladie de Lyme. La méthode de détection, telle que nous la proposons, basée sur l'hybridation moléculaire entre l'ADN cible et une sonde, ou une paire d'amorces originale, spécifique à Bb permet d'assurer ce type d'objectif.

### F. Suivi de la composition des vaccins.

La validité d'un vaccin est déterminée par son pouvoir de protection vis-à-vis de l'agent pathogène cible. Dans le cas spécifique de Bb, le vaccin peut être développé en essayant de dresser une immunité contre la glycoprotéine de surface OspA. Une bonne connaissance de la séquence de ce gène provenant d'un grand nombre de souches différentes appartenant à des groupes différents aide à l'édification d'un tel type de vaccin. Un certain nombre d'auteurs ont indiqué une variabilité du gène OspA. La raison de cette variabilité n'est pas encore connue.

La variabilité du répertoire génétique général de Bb, la variabilité de la séquence du gène OspA et l'état de la connaissance des différentes souches de Bb nécessite une haute vigilance quant à la construction de ce type de vaccin. L'analyse d'un grand nombre d'isolats différents selon les méthodes originales d'investigation moléculaire telles que nous le proposons permet de déterminer le champs d'application de ce type de vaccin. L'utilisation combinée de sondes originales (ou paires d'amorces) spécifiques à chacun ou à tous les groupes de souches de Bb permettra de classer les isolats étudiés dans un des groupes connus dans l'état actuel de la connaissance ou de la positionner dans un des nouveaux groupes créés à cet effet.

L'invention sera maintenant décrite plus concrètement dans les exemples qui suivent:

### Exemple 1.

### A. Détermination des séquences oligonucléotidiques communes à tous les groupes de Borrelia burgdorferi.

Un des buts de la présente invention est de proposer des séquences communes à tous les groupes de souches de Bb, utilisables soit comme amorces au cours de réactions PCR, soit comme sonde de capture dans un test d'hybridation sur support solide où l'ADN cible est pris en "sandwich" entre cette sonde de capture et une sonde de révélation, soit comme sondes de révélation dans ce test d'hybridation de type "sandwich" ou dans un test de détection directe.

L'utilisation des programmes de comparaison de séquences ("Pileup" et "Pretty") distribués par le Genetics Computer Groups, Inc. (University Research Park, 575 Science Drive, Suite B. Madison, Wisconsin 53711, USA) permet de caractériser ce type de séquence. Le programme informatique "Pileup" permet l'alignement de plus de 300 séquences dont la longueur peut atteindre 5.000 nucléotides. Ce programme dérive d'une méthode d'alignement progressif développée par Feng et Doolittle [9]. Le programme "Pretty" conduit à l'alignement des séquences étudiées et à la création d'une séquence "consensus" à partir de cet alignement. A partir de ce mode de présentation des séquences étudiées, il est possible de déterminer rapidement et aisément les séquences cibles communes à tous les groupes de souches de Bb qui peuvent jouer le rôle d'amorces dans une réaction PCR, de sondes dans un système de détection directe ou indirecte ou encore de sonde de capture dans un test d'hybridation de type "sandwich". Ces séquences nucléotidiques possèdent une très haute homologie avec la séquence cible.

Les séquences oligonucléotidiques utilisables comme sondes communes à tous les groupes de souches de Bb sont présentées dans le tableau 2 ci-dessous. La séquence de ces oligomères, orientée de l'extrémité 5' vers l'extrémité 3', se situe sur le brin codant du gène OspA de la souche B31.

**Tableau 2**

| Sondes | Séquences | Taille | Position |
|---|---|---|---|
| Bb50 | ATGAAAAAATATTTATTGGGAATAGGTCTAATATTA GCCTAATAGCATG | 50 | 1 - 50 |
| Bb35bio | GCAACAGTAGACAAGCTTGAGCTTAAAGGAACTTC | 35 | 166 - 200 |
| Bb23bio | TTTTCAAAGA AGATGGCAAA ACA | 23 | 302 - 324 |

Les séquences oligonucléotidiques utilisables comme amorces communes à tous les groupes de souches de Bb, la longueur de ces amorces, leur température de demi-hybridation (Tm) et leur position par rapport à la séquence codante du gène OspA de la souche B31 sont présentées dans le tableau 3 ci-dessous. La séquence de ces oligomères, orientée de l'extrémité 5' vers l'extrémité 3', se situe sur le brin codant pour l'amorce OspApc1; elle se situe sur le brin non codant pour l'amorce OspApc2. La séquence de l'oligomère OspApc2 est présentée en mode complémentaire inverse à la séquence située sur le brin codant.

**Tableau 3**

| Sondes | Séquences | Long. | Tm | Position |
|---|---|---|---|---|
| OspApc1 | AATAGGTCTA ATATTAGCCT TAATAGC | 27 | 70°C | 21 - 47 |
| OspApc2 | CTAGTGTTTT GCCATCTTCT TTGAAAA | 27 | 70°C | 328 - 302 |

Les séquences dans ces sondes ou amorces présentent un très haut pourcentage d'homologie aux séquences connues du gène OspA de toutes les souches de Bb. Ce haut pourcentage d'homologie (98-100%) garantit des hybridations ou amplifications hautement spécifiques à toutes les souches de Bb.

### B. Détermination de séquences d'ADN spécifiques à chaque groupe de souches de Borrelia burgdorferi.

Un autre but de la présente invention est de proposer des séquences nucléotidiques spécifiques à chaque groupe de souches de Bb, utilisables soit comme amorces au cours dune réaction d'amplification génétique (PCR), soit comme sonde de révélation ou de capture dans un test d'hybridation de type "sandwich" ou dans un test de détection directe.

Le traitement informatique ("Pileup" et "Pretty") des séquences disponibles du gène OspA des différentes souches de Bb permet de déterminer aisément les séquences susceptibles d'être utilisées comme sonde ou amorce spécifique à chacun des groupes connus de souches de Bb.

Les séquences oligonucléotidiques de telles sondes, leur position, leur longueur et leur température de demi-hybridation sont présentées dans le tableau 4 ci-dessous. La position des oligomères BbGI23, BbGII23 et BbGIII28 se situe respectivement sur le brin codant du gène OspA de la souche B31, B29 et AcaI.

**Tableau 4**

| Sondes | Séquences | Position | Taille | Tm |
|---|---|---|---|---|
| BbGI23 | CTGCAGCTTGGAATTCAGGCACT | 638 - 660 | 23 | 70°C |
| BbGII23 | ACTCTAGCTGCTGACGGCAAAAC | 508 - 530 | 23 | 70°C |
| BbGIII28 | AGGAAAAGTAGCTAATGATAAAGTAACA | 503 - 530 | 28 | 70°C |

Les séquences oligonucléotidiques des amorces spécifiques à chacun des groupes de souches de Bb, leur position, leur taille et leur température de demi-hybridation sont présentées dans le tableau 5 ci-dessous. Les séquences de telles amorces sont orientées de l'extrémité 5' vers l'extrémité 3'. La position des oligomères BbGI1 et BbGI2 se réfère au brin codant du gène OspA de la souche B31 (groupe I); celle des oligomères BbGII1 et BbGII2 se réfère à la séquence codante du gène OspA de la souche B29 (groupe II); et enfin, celle des oligomères BbGIII1, BbGIII2 et BbGIII3 se rapporte à la séquence codante du gène OspA de la souche AcaI (groupe III). Les séquences des oligomères BbGI2, BbGII2 et BbGIII2 sont présentées en mode complémentaire inverse à la séquence située sur le brin codant.

**Tableau 5**

| Amorces | Séquences | Position | Taille | Tm |
|---|---|---|---|---|
| BbGI1 | AACAAAGACGGCAAGTACGATCTAATT | 139 - 165 | 27 | 74°C |
| BbGI2 | TTACAGTAATTGTTAAAGTTGAAGTGCC | 682 - 655 | 28 | 74°C |
| BbGII1 | TGATAAAAACAACGGTTCTGGAAC | 201 - 224 | 24 | 66°C |
| BbGII2 | GTAACTTTCAATGTTGTTTTGCCG | 545 - 522 | 24 | 66°C |
| BbGIII1 | GAAAAAGGTGAATTGTCTGCAAAAACC | 381 - 407 | 27 | 74°C |
| BbGIII2 | TTCCAATGTTACTTTATCATTAGCTACTT | 536 - 508 | 29 | 74°C |
| BbGIII3 | TAAAGACAAAACATCAACAGATGAAATG | 347 - 374 | 28 | 72°C |

Les séquences de ces sondes ou amorces spécifiques de groupe présentent une homologie à 100% aux séquences connues du gène OspA des groupes cibles de souches de Bb. Ce haut pourcentage d'homologie garantit des hybridations ou amplifications hautement spécifiques aux différents groupes de souches de Bb.

### Example 2.

### Vérification expérimentale de la qualité des séquences communes à tous les groupes de Borrelia burgdorferi.

### A. Hybridation de la sonde Bb23bio à l'ADN de 3 souches représentatives des différents groupes connus de souches Bb.

L'ADN des souches B31 (groupe 1), ZQ1 (groupe II) et AcaI (groupe III) a été préparé selon les méthodes développées par Lefebvre et coli. [10] et par Simpson et coli. [11]. Des quantités variables (de 1 µg à 100 ng) d'ADN issus de ces 3 souches (B31, ZQ1 et AcaI) ont été déposées sur membrane de nylon (Hybond N, Amersham) en utilisant l'appareil Bio-Dot^{R} fourni par la Société BioRad. Les ADN ont été ensuite fixés à la membrane par un traitement de 3 minutes aux ultraviolets (longueur d'onde: 254 nm). La membrane a été pré-hybridée et hybridée selon le protocole présenté par la Société Tropix (Tropix Inc., 47 Wiggins Avenue, Bedford, Massachussets 01730, USA). Ce protocole comprend une incubation de la membrane pendant 1 heure à la température de 37°C dans une solution d'hybridation (1mM EDTA, 7% (P/V) SDS, 0,25 M phosphate disodique, pH7,2, 1% I-Block™). La membrane est ensuite hybridée dans cette solution pendant 1 nuit à la température de 42°C en présence de la sonde Bb23bio à la concentration de 50 ng par ml de solution d'hybridation. La sonde Bb23bio a été modifiée chimiquement par l'addition d'une molécule, par exemple, la biotine, ayant une affinité pour un complexe moléculaire constitué d'une partie enzymatique, par exemple, la phosphatase alcaline.

Après l'hybridation, la membrane a été lavée successivement par des solutions composées soit de 2*SSC/SDS 1% (0,3 M Sodium citrate/0,03 M Sodium chloride/1% sodium dodecylsulfate); soit de 1*SSC/SDS 1%; et enfin, soit de 1*SSC. Chaque lavage a été réalisé à la température de 42°C pendant 2*10 minutes.

Les hybrides moléculaires, ADN cible-sonde, ont été détectés par chimiluminescence tel qu'il est décrit dans le protocole d'utilisation du système de détection "Southern-Light™, Chemiluminescent Detection System" développé par la Société Tropix.

Il résulte que la sonde Bb23bio reconnaît l'ADN des 3 souches analysées (B31, ZQ1 et AcaI). La sensibilité du système permet la détection de ± 100 ng d'ADN (équivalent à 100 10⁶ copies d'ADN de Borrelia burgdorferi).

### B. Amplification génétique (PCR) de fragments d'ADN caractéristiques à tous les groupes connus de souches de Bb par les amorces OspApc1 et OspApc2.

La présente invention a également pour objet des amorces oligonucléotidiques utilisables pour l'amplification de fragments d'ADN caractéristiques à toutes les souches de Bb. Les amorces OspApc1 et OspApc2 permettent d'amplifier un fragment d'ADN de 282 paires de bases caractéristiques à toutes les souches de Bb. Pour obtenir ce fragment d'ADN, la réaction d'amplification génétique est réalisée sur 100 ng d'ADN préparé selon les méthodes développées par Lefebvre et coll. [9] et par Simpson et coll. [10]. Cette réaction PCR est menée en deux phases:
- une phase de dénaturation, de l'ADN cible, de 10 minutes à 96°C.
- une phase d'amplification comprenant 3 étapes répétées 35 fois. La première étape permet l'hybridation des amorces à l'ADN cible; la deuxième étape réalisée à 72°C assure la synthèse d'un ADN complémentaire à l'ADN cible par l'utilisation de l'enzyme de polymérisation, la Taq polymérase; et enfin, la troisième étape permet la dissociation des hybrides formés au cours des deux premières étapes.

Dans le cadre d'utilisation des amorces OspApc1 et OspApc2 dans ce type de réaction, l'étape d'hybridation a été menée à 60°C pendant 1 minute, celle de polymérisation a été réalisée à 72°C pendant 2 minutes et l'étape de dissociation des hybrides a été accomplie à 94°C pendant 1 minute.

Les ADNs de 3 souches (B31, ZQ1 et AcaI) représentatives de chacun des groupes de souches de Bb ont été analysés selon la méthode PCR indiquée ci-dessus. Un dixième de la réaction PCR a été analysé sur gel d'agarose contenant du Nusieve à 3% et du SeaKem à 1%. Il résulte qu'un fragment de 282 paires de bases est obtenu pour les 3 souches analysées après électrophorèse, coloration du gel au bromure d'éthidium et visualisation sur un transluminateur U.V. Ceci indique la bonne qualité des amorces OspApc1 et OspApc2 à être utilisées dans une réaction PCR ainsi que leur possibilité de reconnaître des souches appartenant aux 3 groupes connus de souches de Bb.

### Exemple 3.

### Vérification expérimentale de la qualité des séquences spécifiques à chacun des groupes connus de souches de Borrelia burgdorferi.

### A. Hybridation des sondes spécifiques de groupes (BbGI23, BbGII23 et BbGIII28) à l'ADN de 3 souches représentatives des différents groupes connus de souches de Bb.

La procédure de préparation de l'ADN de 3 souches représentatives des différents groupes connus de souches (B31 pour le groupe I, ZQ1 pour le groupe II et AcaI pour le groupe III) de Bb et la mise en évidence des hybrides moléculaires, ADN cible-sonde (BbGI23 ou BbGII23 ou BbGIII28) ont été menées dans des conditions identiques à celles développées dans le test d'hybridation directe mis au point pour les sondes communes a tous les groupes connus de souches de Bb (voir Exemple 2 - point A).

Il résulte à la suite de ces procédures expérimentales que la sonde BbGI23 caractéristique des souches du groupe I reconnaît spécifiquement l'ADN de la souche B31 (souche du groupe I) à la température d'hybridation de 45°C-50°C. A cette température d'hybridation, la sonde BbGI23 ne reconnaît pas l'ADN issu des souches ZQ1 (groupe II) et AcaI (groupe III).

Il résulte aussi que la sonde BbGII23 caractéristique des souches du groupe II reconnaît spécifiquement l'ADN de la souche ZQ1 (souche du groupe II) à la température d'hybridation de 45°C-50°C. A cette température d'hybridation, la sonde BbGII23 ne reconnaît pas l'ADN issu des souches B31 (groupe I) et AcaI (groupe III).

Il résulte également à la suite de ces procédures expérimentales que la sonde BbGIII28 caractéristique des souches du groupe III reconnaît spécifiquement l'ADN de la souche AcaI (souche du groupe III) à la température d'hybridation de 45°C-50°C. A cette température d'hybridation, la sonde BbGIII28 ne reconnaît pas l'ADN issu des souches B31 (groupe I) et ZQ1 (groupe II).

L'ensemble de ces résultats est repris dans le tableau 6 ci-dessous:

**Tableau 6**

| Sondes | B31 | ZQ1 | AcaI |
|---|---|---|---|
| BbGI23 | + | - | - |
| BbGII23 | - | + | - |
| BbGIII28 | - | - | + |

### B. Amplification génétique (PCR) de fragments d'ADN spécifiques à chacun des groupes connus de souches de Bb.

La présente invention a également pour objet des amorces oligonucléotidiques utilisables pour l'amplification de fragments d'ADN spécifiques de chacun des groupes connus de souches de Bb. Les amorces BbGI1 et BbGI2 permettent d'amplifier un fragment d'ADN de 544 paires de bases; l'utilisation des oligomères BbGII1 et BbGII2 permet une amplification d'un fragment d'ADN de 345 paires de bases; la paire d'oligomères BbGIII3 et BbGIII2 génère après amplification un fragment d'ADN de 188 pb. Pour obtenir ces fragments d'ADN, la réaction d'amplification génétique est réalisée sur 100 ng d'ADN, à partir de différentes souches (B31, ZQ1 et AcaI) représentatives des différents groupes connus de souches de Bb. L'ADN de ces souches est préparé selon les méthodes décrites dans l'exemple 2 point A. La réaction PCR a été menée en deux phases telles qu'elles sont décrites dans l'exemple 2 point B, avec la différence que la température d'hybridation pour la paire GI1/GI2 a été de 69°C; pour la paire GII1/GII2, cette température d'hybridation a été de 68°C; et enfin, pour la paire GIII2/GIII3, la température d'hybridation a été de 68°C.

L'analyse des produits de réaction PCR par gel d'électrophorèse indique que la paire GI1/G12 spécifique des souches du groupe I permet, à la température de 69°C, l'amplification d'un fragment d'ADN de 544 paires de bases uniquement à partir d'ADN issu de la souche B31 (groupe I). L'utilisation de cette paire d'amorces on présence d'ADN issu des souches ZQ1 (groupe II) et AcaI (groupe III) ne permet pas l'amplification de ce fragment de 544 paires de bases.

Cette analyse indique également que la paire GII1/GII2 spécifique des souches du groupe II permet, à la température de 68°C, l'amplification d'un fragment d'ADN de 345 paires de bases uniquement à partir d'ADN issu de la souche ZQ1 (souche du groupe II). L'utilisation de cette paire d'amorces en présence d'ADN issu des souches B31 (groupe I) et AcaI (groupe III) ne permet pas l'amplification de ce fragment de 345 paires de bases.

Il résulte aussi que la paire GIII3/GII2 spécifique des souches du groupe III permet, à la température de 70°C, l'amplification d'un fragment d'ADN de 188 paires de bases uniquement à partir d'ADN issu de la souche AcaI (souche du groupe III). L'utilisation de cette paire d'amorces on présence d'ADN issu des souches B31 (groupe I) et ZQ1 (groupe II) ne permet pas l'amplification de ce fragment de 188 paires de bases.

L'ensemble de ces résultats est repris dans le tableau 7 ci-dessous:

**Tableau 7**

| Amorces | B31 | ZQ1 | AcaI |
|---|---|---|---|
| BbGI1/BbGI2 | + | - | - |
| BbGII1/BbGII2 | - | + | - |
| BbGIII3/BbGIII2 | - | - | + |

Ces résultats déterminent clairement la spécificite et la validité de ces amorces. En effet, la paire d'amorces (BbGI1/BbGI2) spécifique du groupe I ne reconnaît que la souche du groupe I; de même, les paires d'amorces BbGII1/BbGII2 et BbGIII3/BbGIII2 ne reconnaissent respectivement que les souches appartenant au groupe II et au groupe III.

### Exemple 4.

### Application du crible à une variété d'isolats de souches de Borrelia burgdorferi. Classification - Etude par amplification génétique (PCR).

Les études d'hybridation moléculaire sur génome entier de Bb ont permis de séparer en 3 grands groupes, un grand nombre de souches connues. Cette répartition en différents groupes est également mise en évidence en comparant la séquence du gène codant pour la glycoprotéine de surface OspA de différentes souches de Bb par un programme informatique adapté. Les résultats de cette analyse informatique sont corroborés par une étude phylogénétique menée sur le gène codant pour l'ARN ribosomal 16S. L'analyse de cette classification des souches de Bb en relation aux signes cliniques observés au cours de la maladie suggèrent un lien intéressant entre l'appartenance d'une souche à un groupe déterminé et la pathologie qui y est associée; par exemple, les souches reprises dans le groupe I montrent une propension à provoquer des signes arthritiques tandis que celles appartenant au groupe II provoquent principalement des neuroborélioses. Les souches du groupe III provoquent essentiellement des acrodermatites.

Différentes souches de Bb, classées ou non par les études d'hybridation moléculaire sur génome entier, ont été analysées par la méthode de PCR en utilisant les amorces spécifiques de groupes définies dans la section 2.A. - Séquences spécifiques de groupes utilisables au cours de réactions d'amplification génétique (PCR). Les résultats de cette analyse sont développés dans le tableau 8 ci-dessous où BbGI, BbGII, BbGIII et OspA représentent respectivement les pures d'amorces BbGI1/BbGI2 (spécifiques des souches du groupe I), BbGII1/BbGII2 (spécifiques des amorces du groupe II), BbGIII1/BbGIII2 (spécifiques des amorces du groupe III) et OspApc1/OspApc2 (caractéristiques de toutes les souches de Bb).

**Tableau 8**

| **Groupe** | **Souches** | **BbGI** | **BbGII** | **BbGIII** | **OspA** |
|---|---|---|---|---|---|
| **I** | IP1 | ++ | - | - | + |
| | IP2 | ++ | - | - | + |
| | IP3 | ++ | - | - | + |
| | 297 master | ++ | - | - | + |
| | IRS | ++ | - | - | + |
| | 21.038 | ++ | - | - | + |
| | ZS7 | ++ | - | - | + |
| | **B31** | **++** | **-** | **-** | **+** |
| **II** | **ZQ1** | **-** | **++** | **-** | **+** |
| | **Ne11H** | **-** | **+** | **-** | **+** |
| | N34 | - | + | - | + |
| | P/Bi | - | + | - | + |
| **III** | **AcaI** | **-** | **-** | **++** | **+** |
| **IV** | 20047 | - | - | - | + |
| | G25 | + | ++ | ++ | + |
| | VS461 | + | - | ++ | + |

Il résulte de cette analyse qu'un grand nombre de souches peuvent être classées dans un des groupes connus de souches de Bb. Cette classification par analyse des produits de réaction de PCR se superpose en partie à la classification établie par le groupe de Baranton et coll [6]. En effet, certaines souches (FR47, G25 et VS461) considérées par le groupe de Baranton comme étant des souches appartenant au groupe II (FR47 et G25) ou au groupe III (VS461) ne peuvent être classées dam ces groupes par simple analyse des produits de réaction de PCR. Le comportement de ces souches (FR47, G25 et VS461) vis-à-vis des amorces spécifiques de groupes conduit à la création d'un quatrième groupe de souches de Bb. Le développement de ce type d'analyse (PCR) sur un plus grand nombre d'isolats de Bb et l'étude de la séquence du gène OspA des souches du groupe IV permettront de déterminer la validité de la classification reprise dam le tableau 8 ci-dessus et conduiront éventuellement à la création de nouveaux groupes de souches de Bb.

### Exemple 5.

### Applications à des spécimens cliniques (humains et animaux).

Le spirochète Bb s'installe très rapidement dans différents organes et tissus cibles de l'hôte (vessie, système nerveux central, tissus cartilagineux) après avoir transité par la sang et le foie. Il est donc intéressant d'analyser soit par PCR soit par un système d'hybridation moléculaire directe ou de type "sandwich" les liquides biologiques infectés préférentiellement par Bb.

Après préparation rapide (lavage, centrifugation et traitement à la chaleur) de l'ADN d'une série de liquides biologiques (liquide articulaire, liquide céphalorachidien, serum urine) provenant de patients sains ou ayant développé une maladie de Lyme. l'ADN de ces échantillons été soumis à une amplification génétique en utilisant soit les amorces spécifiques de groupes soit les amorces communes à tous les groupes de souches de Bb. Les résultats de ces analyses sont repris dans le tableau 9 ci-dessous:

**Tableau 9**

| Echantillon | OspA | BbGI | BbGII | BbGIII |
|---|---|---|---|---|
| LCR1 | + | ND⁽¹⁾ | ND | ND |
| LCR2 | - | ND | ND | ND |
| LCR- | - | - | - | ND |
| LAR1 | + | - | + | ND |
| LAR2 | + | - | + | ND |
| LAR3 | - | - | - | ND |
| UR1 | + | ND | ND | ND |

| | | | | |
|---|---|---|---|---|
| (1) ND = non déterminé | | | | |

Les résultats de ces réactions PCR permettent de confirmer le diagnostic clinique (LAR1, LAR2, UR1, LCR-) ou d'affiner celui-ci (LCR1 et LCR2: négatifs en sérologie). Ces résultats permettent de démontrer l'efficacité du traitement administré au patient (LAR3, LCR3 à 8). Ils permettent aussi d'indiquer le type de souches de Bb infectant les patients (LAR1 et LAR2: groupe II).

Dans la description qui précède, il est entendu que l'invention couvre également les séquences complémentaires inverses des sondes Bb50, Bb35bio, Bb23bio, BbGI23, BbGII23 et BbGIII28.

### Bibliographie:

[1] Barbour, A.G., and S.F. Hayes, 1986, Miciobiol. Rev. 50, 381 - 400.
[2] Szczepanski, A., and J.L Benach. 1991. Miciobiol. Rev. 55, 21 - 34.
[3] Lonneux, J.F., Van Impe, G., Lebrun, Ph., Tricot, J.-M., et B., Losson. 1990. Rev. Quest. Scient. 161, 189 - 208.
[4] Saiki, R., Scharf, S., Faloona, F., Mullis, K.B., Horn, G.T., Erlich, H.A., and N., Arnheim. 1985. Scient. 230, 1350 - 1354.
[5] Marconi, R.T., and C.F., Garon. 1992. J. Clin. Microbiol. 174, 241 - 244.
[6] Baranton, G., Postic, D., Saint-Girons, I., Boerlin, P., Piffaretti, J.C., Assores, M., and P.A. Grimont. 1992. Int. J. Syst. Bacteriol. 42, 378-383.
[7] Assous, M.V., Postic, D., Paul, G., Névot, P., and G., Baranton. 1992. V International Conference on Lyme Borreliosis, Abstract 06.
[8] Hansen, K., Hindersson, P., and N.S., Pedersen. 1988. J. Clin. Microbiol. 26, 338 - 346.
[9] Feng and Doolittle. 1987. J. Mol. Evol. 35, 351 - 360.
[10] Lefebvre, R.B., Land, R.S., Pergn, G.-C., Brown, J.A., and R.C., Johnson. 1990. J. Clin. Microb. 28, 700 - 707.
[11] Simpson, W.J.. Garon, C.F., and T.G., Schwan. 1990. Infec. Imm. 58, 847 - 853.

## Revendications

1. Agent oligonucléotidique utilisable pour la mise en évidence d'au moins une souche de Borrelia burgdorferi (Bb) par hybridation moléculaire, caractérisé en ce qu'il comprend au moins un oligomère choisi parmi:
| Références | Séquences |
|---|---|
| OspApc1 | AATAGGTCTA ATATTAGCCT TAATAGC |
| OspApc2 | CTAGTGTTTTGCCATCTTCT TTGAAAA |
| Bb50 | 5'-ATGAAAAAATATTT ATTGGGAATAGGTCTA ATATTAGC CTTAATAGCATG-3' |
| Bb35bio | 5'-GCAACAGTAG ACAAGCTTGA GCTTAAAGGA ACTTC-3' |
| Bb23bio | 5'-TTTTCAAAGA AGATGGCAAA ACA-3' |
| BbGI1 | 5'-AACAAAGACG GCAAGTACGA TCTAATT-3' |
| BbGI2 | 5'-TTACAGTAAT TGTTAAAGTT GAAGTGCC-3' |
| BbGII1 | 5'-TGATAAAAAC AACGGTTCTG GAAC-3' |
| BbGII2 | 5'-GTAACTTTCA ATGTTGTTTT GCCG-3' |
| BbGIII1 | 5'-GAAAAAGGTG AATTGTCTGC AAAAACC-3' |
| BbGIII2 | 5'-TTCCAATGTT ACTTTATCAT TAGCTACTT-3' |
| BbGIII3 | 5'-TAAAGACAAA ACATCAACAG ATGAAATG-3' |
| BbGII23 | 5'-ACTCTAGCTG CTGACGGCAA AAC-3' |
| BbGIII28 | 5'-AGGAAAAGTA GCTAATGATA AAGTAACA-3' |
étant entendu que A, T, C, G représentent les nucléotides correspondant aux bases adénine, thymine, cytosine et guanine respectivement, que les séquences du tableau ci-dessus sont présentées dans l'orientation 5' → 3', que les séquences OspApc2, BbGI2, BbGII2 et BbGIII2 sont présentées en mode complémentaire inverse à la séquence du brin codant.

2. Agent oligonucléotidique suivant la revendication 1, comprenant une paire d'amorces d'acides nucléiques utilisables pour l'amplification génétique de l'ADN de tous les groupes de souches connues de Bb, dont l'une est située sur le brin codant et l'autre sur le brin complémentaire en aval de la première amorce, caractérisé en ce qu'il comprend la paire d'amorces suivante:
| **Sondes** | **Séquences** | **Long.** | **Tm** | **Position** |
|---|---|---|---|---|
| OspApc1 | AATAGGTCTA ATATTAGCCT TAATAGC | 27 | 70°C | 21 - 47 |
| OspApc2 | CTAGTGTTTT GCCATCTTCT TTGAAAA | 27 | 70°C | 328 - 302 |
étant entendu que A, T, C et G ont les significations données précédemment, que le tableau ci-dessus présente la position desdites amorces sur la séquence connue du gène OspA de la souche B31 de Bb, leur température de demi-hybridation et leur taille respective, que les séquences sont présentées dans l'orientation 5' → 3' et que la séquence de l'amorce OspApc2 est présentée en mode complémentaire inverse à la séquence du brin codant.

3. Agent oligonucléotidique suivant la revendication 1, utilisable comme sonde commune à tous les groupes de souches de Bb, caractérisé en ce qu'il comprend un oligomère choisi parmi les suivants et ceux de séquences complémentaires inverses:
| **Sondes** | **Séquences** | **Taille** | **Position** |
|---|---|---|---|
| Bb50 | ATGAAAAAATATTTATTGGGAATAGGTCTAATATTA GCCTTAATAGCATG | 50 | 1 - 50 |
| Bb35bio | GCAACAGTAGACAAGCTTGAGCTTAAAGGAACTTC | 35 | 166 - 200 |
| Bb23bio | TTTTCAAAGA AGATGGCAAA ACA | 23 | 302 - 324 |
étant entendu que A, T, C et G ont les significations données précédemment, que le tableau ci-dessus présente la taille et la position desdites séquences orientées de l'extrémité 5' vers l'extrémité 3' sur le brin codant du gène OspA de la souche B31 de Bb, dont la séquence est connue.

4. Agent oligonucléotidique suivant la revendication 3, utilisable pour la capture de l'ADN issu de tous les groupes de souches de Bb, caractérisé en ce qu'il comprend l'oligomère Bb50 tel que défini précédemment ou un oligomère de séquence complémentaire inverse.

5. Agent oligonucléotidique suivant la revendication 3, utilisable comme sonde de révélation d'une réaction d'hybridation entre une sonde de capture et un ADN cible, cette sonde de révélation étant modifiée chimiquement par l'addition en 5' et/ou an 3' d'un ou plusieurs groupements permettant la révélation par coloration ou chimiluminescence, caractérisé en ce qu'il comporte une séquence d'acides nucléiques choisie parmi les oligomères Bb35bio et Bb23bio tels que définis précédemment et les oligomères de séquences complémentaires inverses.

6. Agent oligonucléotidique suivant la revendication 1, comprenant une paire d'amorces d'acides nucléiques utilisables pour l'amplification génétique des souches de Bb du groupe I (notamment la souche B31), caractérisé an ce qu'il comprend la paire d'amorces suivante:
| **Références** | **Séquences** | **Position** | **Taille** | **Tm** |
|---|---|---|---|---|
| BbGI1 | AACAAAGACGGCAAGTACGATCTAATT | 139 - 165 | 27 | 74°C |
| BbGI2 | TTACAGTAAT TGTTAAAGTT GAAGTGCC | 682 - 655 | 28 | 74°C |
étant entendu que A, T, C et G ont les significations données précédemment, que le tableau ci-dessus présente la position desdites amorces sur la séquence connue du gène OspA de la souche B31 de Bb en se référant au brin codant dudit gène, leur température (Tm) de demi-hybridation et leur taille respective, que les séquences sont présentées dans l'orientation 5' → 3' et que la séquence de l'amorce BbGI2 est présentée en mode complémentaire inverse à la séquence située sur le brin codant.

7. Agent oligonucléotidique suivant la revendication 1, comprenant une paire d'amorces d'acides nucléiques utilisables pour l'amplification génétique des souches de Bb du groupe II (notamment la souche B29), caractérisé en ce qu'il comprend la paire d'amorces suivante:
| **Références** | **Séquences** | **Position** | **Taille** | **Tm** |
|---|---|---|---|---|
| BbGII1 | TGATAAAAACAACGGTTCTGGAAC | 201 - 224 | 24 | 66°C |
| BbGII2 | GTAACTTTCAATGTTGTTTTGCCG | 545 - 522 | 24 | 66°C |
étant entendu que A, T, C et G ont les significations données précédemment, que le tableau ci-dessus présente la position desdites amorces sur la séquence connue du gène OspA de la souche B29 de Bb en se référant au brin codant dudit gène, leur température (Tm) de demi-hybridation et leur taille respective, que les séquences sont présentées dans l'orientation 5' → 3' et que la séquence de l'amorce BbGII2 est présentée en mode complémentaire inverse à la séquence située sur le brin codant.

8. Agent oligonucléotidique suivant la revendication 1, comprenant une paire d'amorces d'acides nucléiques utilisables pour l'amplification génétique des souches de Bb du groupe III (notamment la souche AcaI), caractérisé en ce qu'il comprend les amorces suivantes mises en oeuvre par paires:
| **Références** | **Séquences** | **Position** | **Taille** | **Tm** |
|---|---|---|---|---|
| BbGIII1 | GAAAAAGGTGAATTGTCTGCAAAAACC | 381 - 407 | 27 | 74°C |
| BbGIII2 | TTCCAATGTTACTTTATCATTAGCTACTT | 536 - 508 | 29 | 74°C |
| BbGIII3 | TAAAGACAAAACATGAACAGATGAAATG | 347 - 374 | 28 | 72°C |
étant entendu que A, T, C et G ont les significations données précédemment, que le tableau ci-dessus présente la position desdites amorces sur la séquence connue du gène OspA de la souche AcaI de Bb en se référant au brin codant dudit gène, leur température (Tm) de demi-hybridation et leur taille respective, que les séquences sont présentées dans l'orientation 5' → 3' et que la séquence de l'amorce BbGIII2 est présentée en mode complémentaire inverse à la séquence située sur le brin codant.

9. Agent oligonucléotidique suivant la revendication 1, comprenant une sonde d'acides nucléiques spécifiques aux souches de Bb du groupe II (notamment la souche B29), soit pour la détection directe soit pour la détection indirecte via un système d'hybridation moléculaire dit "sandwich", caractérisé en ce qu'il comprend l'oligomère:
| **Référence** | **Séquence** | **Position** | **Taille** | **Tm** |
|---|---|---|---|---|
| BbGII23 | ACTCTAGCTGCTGACGGCAAAAC | 508 - 530 | 23 | 70°C |
ou un oligomère de séquence complémentaire inverse, étant entendu que A, T, C et G ont les significations données précédemment, que le tableau ci-dessus présente la température (Tm) de demi-hybridation, la taille et la position de ladite séquence, orientée de l'extrémité 5' vers l'extrémité 3', sur le brin codant du gène OspA de la souche B29 de Bb, dont la séquence est connue.

10. Agent oligonucléotidique suivant la revendication 1, comprenant une sonde d'acides nucléiques spécifiques aux souches de Bb du groupe III (notamment la souche AcaI), soit pour la détection directe soit pour la détection indirecte via un système d'hybridation moléculaire dit "sandwich", caractérisé en ce qu'il comprend l'oligomère:
| **Référence** | **Séquence** | **Position** | **Taille** | **Tm** |
|---|---|---|---|---|
| BbGIII28 | AGGAAAAGTAGCTAATGATAAAGTAACA | 503 - 530 | 28 | 70°C |
ou un oligomère de séquence complémentaire inverse, étant entendu que A, T, C et G ont les significations données précédemment, que le tableau ci-dessus présente la température (Tm) de demi-hybridation, la taille et la position de ladite séquence, orientée de l'extrémité 5' vers l'extrémité 3', sur le brin codant du gène OspA de la souche AcaI de Bb, dont la séquence est connue.

11. Agent oligonucléotidique suivant l'une quelconque des revendications 9 et 10, lorsqu'il est utilisé pour la détection indirecte via un système d'hybridation moléculaire dit "sandwich" en tant que sonde de capture spécifique au groupe de souches du Bb considéré (respectivement II et III) en combinaison avec une sonde de révélation, laquelle comporte un oligomère Bb35bio ou Bb23bio ou son complémentaire inverse tel que défini à la revendication 3, modifié chimiquement par l'addition en 5' et/ou en 3' d'un ou plusieurs groupements permettant la révélation par coloration ou chimiluminescence.

12. Agent oligonucléotidique suivant l'une quelconque des revendications 9 et 10, lorsqu'il est utilisé, en combinaison avec une sonde de capture commune à tous les groupes des souches de Bb, pour la détection indirecte via un système d'hybridation moléculaire dit "sandwich" en tant que sonde de révélation spécifique au groupe de souches du Bb considéré (respectivement II et III), cette sonde de révélation étant modifiée chimiquement par l'addition en 5' et/ou en 3' d'un ou plusieurs groupements permettant la révélation par coloration ou chimiluminescence.

13. Procédé de détection de Borrelia burgdorferi (Bb) consistant à réaliser un hybride moléculaire entre une sonde de capture et l'ADN du Bb préalablement dénaturé et à mettre en oeuvre une sonde de révélation destinée à révéler la présence de l'hybride moléculaire précité, caractérisé en ce qu'on met en oeuvre une sonde de capture telle que définie à la revendication 4 et une sonde de révélation telle que définie à la revendication 5.

14. Procédé de détection de Borrelia burgdorferi (Bb) consistant à réaliser un hybride moléculaire entre une sonde de capture et l'ADN de Bb préalablement dénaturé et à mettre en oeuvre une sonde de révélation destinée à révéler la présence de l'hybride moléculaire précité, caractérisé en ce qu'on met en oeuvre une sonde de capture spécifique aux souches de Bb de l'un des groupes I, II et III et une sonde de révélation telle que définie à la revendication 5.

15. Procédé suivant la revendication 14, caractérisé en ce que la sonde de capture mise en oeuvre est spécifique aux souches de Bb du groupe I (notamment la souche B31).

16. Procédé suivant la revendication 14, caractérisé en ce que la sonde de capture mise en oeuvre est spécifique aux souches de Bb du groupe II (notamment la souche B29) et est telle que définie à la revendication 9

17. Procédé suivant la revendication 14, caractérisé en ce que la sonde de capture mise en oeuvre est spécifique aux souches de Bb du groupe III (notamment la souche AcaI) est telle que définie à la revendication 10.

18. Procédé de détection de Borrelia burgdorferi (Bb) consistant à réaliser un hybride moléculaire entre une sonde de capture et l'ADN de Bb préalablement dénaturé et à mettre en oeuvre une sonde de révélation destinée à révéler la présence de l'hybride moléculaire précité, caractérisé par la mise en oeuvre d'une sonde de capture commune à toutes les souches de Bb et d'une sonde de révélation spécifique aux souches de Bb de l'un des groupes I, II et III.

19. Procédé suivant la revendication 18, caractérisé en ce que la sonde de révélation est spécifique aux souches de Bb du groupe I (notamment la souche B31).

20. Procédé suivant la revendication 18, caractérisé en ce que la sonde de révélation est spécifique aux souches de Bb du groupe II (notamment la souche B29) et est telle que définie à la revendication 9.

21. Procédé suivant la revendication 18, caractérisé en ce que la sonde de révélation est spécifique aux souches de Bb du groupe III (notamment la souche AcaI) et est telle que définie à la revendication 10.

22. Procédé de détection de Borrelia burgdorferi (Bb) par amplification génétique des fragments d'ADN dénaturé caractéristiques de ce micro-organisme. comprenant l'hybridation d'une paire d'amorces à l'ADN cible, la synthèse d'un ADN complémentaire à l'ADN cible et la dissociation des hybrides formés au cours des deux premières étapes, caractérisé par la mise en oeuvre d'une paire d'amorces utilisables pour l'amplification génétique de l'ADN de tous les groupes de souches connues de Bb, cette paire d'amorces étant définie à la revendication 2.

23. Procédé suivant la revendication 22, caractérisé par la mise en oeuvre d'une paire d'amorces utilisables pour l'amplification génétique des souches de Bb du groupe I (notamment la souche B31), cette paire d'amorces étant définie à la revendication 6.

24. Procédé suivant la revendication 22, caractérisé par la mise en oeuvre d'une paire d'amorces utilisables pour l'amplification génétique des souches de Bb du groupe II (notamment la souche B29), cette paire d'amorces étant définie à la revendication 7.

25. Procédé suivant la revendication 22, caractérisé par la mise en oeuvre d'une paire d'amorces utilisables pour l'amplification génétique des souches de Bb du groupe III (notamment la souche AcaI), cette paire d'amorces étant choisie parmi celles définies à la revendication 8.

26. Utilisation d'un ou plusieurs oligonucléotides tels que définis dans l'une quelconque des revendications 1 à 12, pour la production d'un agent de diagnostic de Borrelia burgdorferi (Bb) dans les échantillons biologiques d'origine humaine ou animale, en particulier pour l'épidémiologie, l'étude des animaux réservoirs, l'aide à la thérapeutique des infections par Bb, le suivi de l'efficacité des vaccinations et la composition des vaccins.

## Claims

1. An oligonucleotide agent which can be used to evidence at least one strain of Borrelia burgdorferi (Bb) by molecular hybridisation, characterised in that it comprises at least one oligomer selected from:
| Designations | Sequences |
|---|---|
| OspApc1 | AATAGGTCTA ATATTAGCCT TAATAGC |
| OspApc2 | CTAGTGTTTTGCCATCTTCT TTGAAAA |
| Bb50 | 5'-ATGAAAAATATTT ATTGGGAATAGGTCTA ATATTAGC CTTAATAGCATG-3' |
| Bb35bio | 5'-GCAACAGTAG ACAAGCTTGA GCTTAAAGGA ACTTC-3' |
| Bb23bio | 5'-TTTTCAAAGA AGATGGCAAA ACA-3' |
| BbGI1 | 5'-AACAAAGACG GCAAGTACGA TCTAATT-3' |
| BbGI2 | 5'-TTACAGTAAT TGTTAAAGTT GAAGTGCC-3' |
| BbGII1 | 5'-TGATAAAAAC AACGGTTCTG GAAC-3' |
| BbGII2 | 5'-GTAACTTTCA ATGTTGTTTT GCCG-3' |
| BbGIII1 | 5'-GAAAAAGGTG AATTGTCTGC AAAAACC-3' |
| BbGIII2 | 5'-TTCCAATGTT ACTTTATCATTAGCTACTT-3' |
| BbGIII3 | 5'-TAAAGACAAA ACATCAACAG ATGAAATG-3' |
| BbGII23 | 5'-ACTCTAGCTG CTGACGGCAA AAC-3' |
| BbGIII28 | 5'-AGGAAAAGTA GCTAATGATA AAGTAACA-3' |
it being understood that A, T, C, G represent the nucleotides corresponding to the bases adenine, thymine, cytosine and guanine, respectively, that the sequences set out in the above Table are presented with the orientation 5'→3', and that the sequences OspApc2, BbGI2, BbGII2 and BbGIII2 are presented in inverse complementary mode to the sequence of the coding strand.

2. The oligonucleotide agent according to claim 1, comprising a pair of nucleic acid primers which can be used for genetic amplification of the DNA of all the groups of known Bb strains, one of which is situated on the coding strand and the other on the complementary strand downstream of the first primer, characterised in that the said oligonucleotide agent comprises the following pair of primers:
| Probes | Sequences | Length | Tm | Position |
|---|---|---|---|---|
| OspApc1 | AATAGGTCTA ATATTAGCCT TAATAGC | 27 | 70°C | 21-47 |
| OspApc2 | CTAGTGTTTT GCCATCTTCT TTGAAAA | 27 | 70°C | 328-302 |
it being understood that A, T, C and G have the meanings given previously, that the above Table sets out the position of the said primers on the known sequence of the OspA gene of Bb strain B31, their respective semi-hybridisation temperature and size, that the sequences are presented in the orientation 5'→3' and that the sequence of the OspApc2 primer is presented in inverse complementary mode to the sequence of the coding strand.

3. The oligonucleotide agent according to claim 1, suited to use as a probe common to all the groups of Bb strains, characterised in that it comprises an oligomer selected from the following list and those with inverse complementary sequences:
| Probes | Sequences | Size | Position |
|---|---|---|---|
| Bb50 | ATGAAAAAATATTTATTGGGAATAGGTCTAATATTA GCCTTAATAGCATG | 50 | 1-50 |
| Bb35bio | GCAACAGTAGACAAGCTT GAGCTTAAAGGAACTTC | 35 | 166-200 |
| Bb23bio | TTTTCAAAGA AGAT GGCAAA ACA | 23 | 302-324 |
it being understood that A, T, C and G have the meanings already given, and that the Table above sets out the size and position of the said sequences oriented from the 5' extremity towards the 3' extremity on the coding strand of the OspA gene of Bb strain B31, the sequence of which is known.

4. The oligonucleotide agent according to claim 3, suited to use in capturing DNA originating from all the groups of Bb strains, characterised in that it comprises the oligomer Bb50 as defined previously or an oligomer of inverse complementary sequence.

5. The oligonucleotide agent according to claim 3, suited to use as a probe for disclosing a hybridisation reaction between a capture probe and a target DNA, the said disclosure probe being modified chemically by the addition at 5' and/or at 3' of one or more groupings that permit disclosure through coloration or chemiluminescence, characterised in that it incorporates a sequence of nucleic acids selected from the oligomers Bb35bio and Bb23bio as defined previously and the oligomers of inverse complementary sequences.

6. The oligonucleotide agent according to claim 1, comprising a pair of nucleic acid primers suited to use in genetic amplification of the Bb strains of group I (notably strain B31), characterised in that it comprises the following pair of primers:
| **Designations** | **Sequences** | **Position** | **Size** | **Tm** |
|---|---|---|---|---|
| BbGI1 | AACAAAGACGGCAAGTACGATCTAATT | 139 - 165 | 27 | 74°C |
| BbGI2 | TTACAGTAAT TGTTAAAGTT GAAGTGCC | 682 - 655 | 28 | 74°C |
it being understood that A, T, C and G have the meanings already given, that the Table above sets out the position of the said primers on the known sequence of the OspA gene of Bb strain B31 by reference to the coding strand of the said gene, their respective semi-hybridisation temperature (Tm) and size, that the sequences are presented in 5'→3' orientation and that the sequence of the BbGI2 primer is presented in inverse complementary mode to the sequence situated on the coding strand.

7. The oligonucleotide agent according to claim 1, comprising a pair of nucleic acid primers suited to use in genetic amplification of Bb strains of group II (notably strain B29), characterised in that it comprises the following pair of primers:
| **Designations** | **Sequences** | **Position** | **Size** | **Tm** |
|---|---|---|---|---|
| BbGII1 | TGATAAAAACAACGGTTCT GGAAC | 201 - 224 | 24 | 66°C |
| BbGII2 | GTAACTTTCAATGTTGTTTTGCCG | 545 - 522 | 24 | 66°C |
it being understood that A, T, C and G have the meanings already given, that the Table above sets out the position of the said primers on the known sequence of the OspA gene of Bb strain B29 by reference to the coding strand of the said gene, their respective semi-hybridisation temperature (Tm) and size, that the sequences are presented in 5'→3' orientation and that the sequence of the BbGII2 primer is presented in inverse complementary mode to the sequence situated on the coding strand.

8. The oligonucleotide agent according to claim 1, comprising a pair of nucleic acid primers suited to use in genetic amplification of the Bb strands of group III (notably strain AcaI), characterised in that it comprises the following primers employed pairwise:
| **Designations** | **Sequences** | **Position** | **Size** | **Tm** |
|---|---|---|---|---|
| BbGIII1 | GAAAAAGGTGAATTGTCTGCAAAAACC | 381 - 407 | 27 | 74°C |
| BbGIII2 | TTCCAATGTTACTTTATCATTAGCTACTT | 536 - 508 | 29 | 74°C |
| BbGIII3 | TAAAGACAAAACATCAACAGATGAAATG | 347 - 374 | 28 | 72°C |
it being understood that A, T, C and G have the meanings already given, that the Table above sets out the position of the said primers on the known sequence of the OspA gene of Bb strain AcaI by reference to the coding strand of the said gene, their respective semi-hybridisation temperature (Tm) and size, that the sequences are presented in 5'→3' orientation and that the sequence of the BbGIII2 primer is presented in inverse complementary mode to the sequence situated on the coding strand.

9. The oligonucleotide agent according to claim 1, comprising a probe of nucleic acids specific to the Bb strains of group II (notably strain B29), either for direct detection or for indirect detection via a so-called "sandwich" system of molecular hybridisation, characterised in that it comprises the oligomer:
| **Designation** | **Sequence** | **Position** | **Size** | **Tm** |
|---|---|---|---|---|
| BbGII23 | ACTCTAGCTGCTGACGGCAAAAC | 508 - 530 | 23 | 70°C |
or an oligomer of inverse complementary sequence, it being understood that A, T, C and G have the meanings already given, that the Table above sets out the semihybridisation temperature (Tm), the size and the position of the said sequence, oriented from the 5' extremity towards the 3' extremity, on the coding strand of the OspA gene of Bb strain B29, the sequence of which is known.

10. The oligonucleotide agent according to claim 1, comprising a probe of nucleic acids specific to the Bb strains of group III (notably the AcaI strain), either for direct detection or for indirect detection via a so-called "sandwich" system of molecular hybridisation, characterised in that it comprises the oligomer:
| **Designation** | **Sequence** | **Position** | **Size** | **Tm** |
|---|---|---|---|---|
| BbGIII28 | AGGAAAAGTAGCTAATGATAAAGTAACA | 503 - 530 | 28 | 70°C |
or an oligomer of inverse complementary sequence, it being understood that A, T, C and G have the meanings already given, that the Table above sets out the semihybridisation temperature (Tm), the size and the position of the said sequence, oriented from the 5' extremity towards the 3' extremity, on the coding strand of the OspA gene of Bb strain AcaI, the sequence of which is known.

11. The oligonucleotide agent according to either of claims 9 and 10, when used for indirect detection via a so-called "sandwich" system of molecular hybridisation as a capture probe specific to the group of Bb strains concerned (II and III respectively) in combination with a disclosure probe which incorporates a Bb35bio or Bb23bio oligomer or its inverse complement as defined in claim 3, chemically modified by the addition at 5' and/or at 3' of one or more groupings that permit disclosure through coloration or chemiluminescence.

12. The oligonucleotide agent according to either of claims 9 and 10, when used, in combination with a capture probe common to all the groups of Bb strains, for indirect detection via a so-called "sandwich" system of molecular hybridisation as a disclosure probe specific to the group of Bb strains concerned (II and III respectively), the said disclosure probe being modified chemically by the addition at 5' and/or at 3' of one or more groupings that permit disclosure through coloration or chemiluminescence.

13. A method for detecting Borrelia burgdorferi (Bb) consisting in realising a molecular hybrid between a capture probe and the DNA of the Bb which has first been denatured and in employing a disclosure probe designed to disclose the presence of the aforesaid molecular hybrid, characterised by employing a capture probe as defined in claim 4 and a disclosure probe as defined in claim 5.

14. A method for detecting Borrelia burgdorferi (Bb) consisting in realising a molecular hybrid between a capture probe and the DNA of the Bb which has first been denatured and in employing a disclosure probe designed to disclose the presence of the aforesaid molecular hybrid, characterised by employing a capture probe specific to the Bb strains of one of groups I, II and III and a disclosure probe as defined in claim 5.

15. The method according to claim 14, characterised in that the capture probe employed is specific to the Bb strains of group I (notably strain B31).

16. The method according to claim 14, characterised in that the capture probe employed is specific to the Bb strains of group II (notably strain B29) and is as defined in claim 9.

17. The method according to claim 14, characterised in that the capture probe employed is specific to the Bb strains of group III (notably strain AcaI) and is as defined in claim 10.

18. A method for detecting Borrelia burgdorferi (Bb) consisting in realising a molecular hybrid between a capture probe and the DNA of the Bb which has first been denatured and in employing a disclosure probe designed to disclose the presence of the aforesaid molecular hybrid, characterised by employing a capture probe common to all strains of Bb and a disclosure probe specific to the Bb strains of one of groups I, II and III.

19. The method according to claim 18, characterised in that the disclosure probe is specific to the Bb strains of group I (notably strain B31).

20. The method according to claim 18, characterised in that the disclosure probe is specific to the Bb strains of group II (notably strain B29) and is as defined in claim 9.

21. The method according to claim 18, characterised in that the disclosure probe is specific to the Bb strains of group III (notably strain AcaI), and is as defined in claim 10.

22. A method of detecting Borrelia burgdorferi (Bb) by genetic amplification of denatured DNA fragments characteristic of that microorganism, comprising the hybridisation of a pair of primers to the target DNA, the synthesis of a DNA complementary to the target DNA and the dissociation of the hybrids formed during the first two stages, characterised by employing a pair of primers suitable for use in genetic amplification of the DNA of all the groups of known Bb strains, the said pair of primers being defined in claim 2.

23. The method according to claim 22, characterised by employing a pair of primers suitable for use in genetic amplification of Bb strains of group I (notably strain B31), the said pair of primers being defined in claim 6.

24. The method according to claim 22, characterised by employing a pair of primers suitable for use in genetic amplification of Bb strains of group II (notably strain B29), the said pair of primers being defined in claim 7.

25. The method according to claim 22, characterised by employing a pair of primers suitable for use in genetic amplification of Bb strains of group III (notably strain AcaI), the said pair of primers being selected from those defined in claim 8.

26. The use of one or more oligonucleotides as defined in any of claims 1 to 12 for the production of a diagnostic agent for Borrelia burgdorferi (Bb) in biological samples of human or animal origin, more particularly for epidemiology, the study of reservoir animals, as an aid to the treatment of infections by Bb, for the follow-up of vaccination efficacy and vaccine composition.

## Patentansprüche

1. Oligonukleotid, verwendbar für den durch molekulare Hybridisierung erfolgenden Nachweis von mindestens einem Stamm von Borrelia burgdorferi (Bb), dadurch gekennzeichnet₁ daß es mindestens eines der nachstehenden Oligomere enthält:
| ***Referenzen*** | ***Sequenzen*** |
|---|---|
| OspApc1 | AATAGGTCTA ATATTAGCCT TAATAGC |
| OspApc2 | CTAGTGTTTTGCCATCTTCT TTGAAAA |
| Bb50 | 5'-ATGAAAAAATATTT ATTGGGAATAGGTCTA ATATTAGC CTTAATAGCATG-3' |
| Bb35bio | 5'-GCAACAGTAG ACAAGCTTGA GCTTAAAGGA ACTTC-3' |
| Bb23bio | 5'-TTTTCAAAGA AGATGGCAAA ACA-3' |
| BbGI1 | 5'-AACAAAGACG GCAAGTACGA TCTAATT-3' |
| BbGI2 | 5'-TTACAGTAAT TGTTAAAGTT GAAGTGCC-3' |
| BbGII1 | 5'-TGATAAAAAC AACGGTTCTG GAAC-3' |
| BbGII2 | 5'-GTAACTTTCA ATGTTGTTTT GCCG-3' |
| BbGIII1 | 5'-GAAAAAGGTG AATTGTCTGC AAAAACC-3' |
| BbGIII2 | 5'-TTCCAATGTT ACTTTATCAT TAGCTACTT-3' |
| BbGIII3 | 5'-TAAAGACAAA ACATCAACAG ATGAAATG-3' |
| BbGII23 | 5'-ACTCTAGCTG CTGACGGCAA AAC-3' |
| BbGIII28 | 5'-AGGAAAAGTA GCTAATGATA AAGTAACA-3' |
mit der Maßgabe, daß A, T, C und G für die Nukleotide entsprechend den Basen Adenin, Thymin, Cytosin bzw. Guanin stehen, daß die Sequenzen der obigen Tabelle in Richtung 5'→ 3' wiedergegeben sind und daß die Sequenzen OspApc2, BbGI2, BbGII2 und BbGIII2 komplementär entgegengesetzt zur Sequenz des codierenden Strangs aufgeführt sind.

2. Oligonukleotid gemäß Anspruch 1, enthaltend ein Paar Primer von Nukleinsäuren, die für die Genamplifikation der DNA aller Gruppen bekannter Stämme von Bb geeignet sind, wovon einer sich auf dem codierenden Strang und der andere auf dem Komplementärstrang stromabwärts des ersten Primers befindet, dadurch gekennzeichnet, daß es das folgende Paar Primer enthält:
| ***Sonden*** | ***Sequenzen*** | ***Länge*** | ***Temperatur*** | ***Position*** |
|---|---|---|---|---|
| OspApc1 | AATAGGTCTA ATATTAGCCT TAATAGC | 27 | 70°C | 21 - 47 |
| OspApc2 | CTAGTGTTTT GCCATCTTCT TTGAAAA | 27 | 70°C | 328 - 302 |
mit der Maßgabe, daß A, T, C und G die zuvor angegebenen Bedeutungen haben, wobei die obige Tabelle die Position der besagten Primer auf der bekannten Sequenz des OspA-Gens des Stamms B31 von Bb, ihre Halb-Hybridisierungstemperatur bzw. ihre Größe wiedergibt, daß die Sequenzen in Richtung 5'→ 3' wiedergegeben sind, und daß die Sequenz des Primers OspApc2 komplementär entgegengesetzt zur Sequenz des codierenden Strangs dargestellt ist.

3. Oligonukleotid gemäß Anspruch 1, verwendbar als allen Gruppen der Stämme von Bb gemeinsame Sonde, dadurch gekennzeichnet, daß es ein Oligomeres enthält, das unter den nachfolgend aufgeführten und denen mit inversen Komplementärsequenzen ausgewählt wurde.
| ***Sonden*** | ***Sequenzen*** | ***Größe*** | ***Position*** |
|---|---|---|---|
| Bb50 | ATGAAAAAATATTTATTGGGAATAGGTCTAATATTA GCCTTAATAGCATG | 50 | 1 - 50 |
| Bb35bio | GCAACAGTAGACAAGCTTGAGCTTAAAGGAACTTC | 35 | 166 - 200 |
| Bb23bio | TTTTCAAAGA AGATGGCAAA ACA | 23 | 302 - 324 |
mit der Maßgabe, daß A, T, C und G die zuvor genannten Bedeutungen haben, daß die obige Tabelle die Größe und Position der besagten Sequenzen wiedergibt, die vom Ende 5' zum Ende 3' auf dem codierenden Strang des Gens OspA des Stamms B31 von Bb ausgerichtet sind, dessen Sequenz bekannt ist.

4. Oligonukleotid gemäß Anspruch 3, verwendbar für das Erfassen der aus allen Gruppen der Stämme von Bb stammenden DNA, dadurch gekennzeichnet, daß es das Oligomere Bb50 wie vorstehend definiert oder ein Oligomeres der inversen Komplementärsequenz enthält.

5. Oligonukleotid gemäß Anspruch 3, verwendbar als Meßsonde einer Hybridisierungsreaktion zwischen einer Erfassungssonde und einer Ziel-DNA, wobei diese Meßsonde durch Zugabe einer oder mehrerer Gruppen bei 5' und/oder 3' chemisch verändert wird und damit die Messung durch Färben oder Chemilumineszenz ermöglicht, dadurch gekennzeichnet, daß es eine Sequenz von Nukleinsäuren enthält, die unter den Oligomeren Bb35bio und Bb23bio wie vorstehend definiert und den Oligomeren der inversen Komplementärsequenzen ausgewählt wird.

6. Oligonukleotid gemäß Anspruch 1, enthaltend ein Paar Primer von Nuklerinsäuren, die verwendbar sind für die Genamplifikation der Stämme von Bb der Gruppe 1 (besonders des Stamms B31), dadurch gekennzeichnet, daß es das folgende Paar Primer enthält:
| ***Referenzen*** | ***Sequenzen*** | ***Position*** | ***Größe*** | ***Temperatur*** |
|---|---|---|---|---|
| BbGI1 | AACAAAGACGGCAAGTACGATCTAATT | 139 - 165 | 27 | 74°C |
| BbGI2 | TTACAGTAAT TGTTAAAGTT GAAGTGCC | 682 - 655 | 28 | 74°C |
mit der Maßgabe, daß A, T, C und G die zuvor genannten Bedeutungen haben, daß die obige Tabelle die Position der besagten Primer auf der bekannten Sequenz des Gens OspA des Stammes B31 von Bb unter Bezug auf den codierenden Strang des besagten Gens, ihre Halb-Hybridisierungstemperatur (Tm) bzw. ihre Größe wiedergibt, daß die Sequenzen in Richtung 5'→ 3' wiedergegeben werden, und daß die Sequenz des Primers BbGI2 komplementär entgegengesetzt zur Sequenz auf dem codierenden Strang dargestellt wird.

7. Oligonukleotid gemäß Anspruch 1, enthaltend ein Paar Primer von Nukleinsäuren, die geeignet sind für die Genamplifikation der Stämme von Bb der Gruppe II (besonders des Stammes B29), dadurch gekennzeichnet, daß es das folgende Paar Primer enthält:
| ***Referenzen*** | ***Sequenzen*** | ***Position*** | ***Größe*** | ***Temperatur*** |
|---|---|---|---|---|
| BbGII1 | TGATAAAAACAACGGTTCTGGAAC | 201 - 224 | 24 | 66°C |
| BbGII2 | GTAACTTTCAATGTTGTTTTGCCG | 545 - 522 | 24 | 66°C |
mit der Maßgabe, daß A, T, C und G die zuvor genannten Bedeutungen haben, daß die obige Tabelle die Position der besagten Primer auf der bekannten Sequenz des Gens OspA des Stammes B29 von Bb unter Bezug auf den codierenden Strang des besagten Gens, ihre HalbHybridisierungstemperatur (Tm) bzw. ihre Größe wiedergibt, daß die Sequenzen in Richtung 5'→ 3' wiedergegeben werden, und daß die Sequenz des Primers BbGII2 komplementär entgegengesetzt zur Sequenz auf dem codierenden Strang dargestellt wird.

8. Oligonukleotid gemäß Anspruch 1, enthaltend ein Paar Primer von Nulkeinsäuren, die geeignet sind für die Genamplifizierung der Stämme von Bb der Gruppe III (besonders des Stammes AcaI), dadurch gekennzeichnet, daß es die folgenden paarweise verwendeten Primer enthält:
| ***Referenzen*** | ***Sequenzen*** | ***Position*** | ***Größe*** | ***Temperatur*** |
|---|---|---|---|---|
| BbGIII1 | GAAAAAGGTGAATTGTCTGCAAAAACC | 381 - 407 | 27 | 74°C |
| BbGIII2 | TTCCAATGTTACTTTATCATTAGCTACTT | 536 - 508 | 29 | 74°C |
| BbGIII3 | TAAAGACAAAACATCAACAGATGAAATG | 347 - 374 | 28 | 72°C |
mit der Maßgabe, daß A, T, C und G die zuvor genannten Bedeutungen haben, daß die obige Tabelle die Position der besagten Primer auf der bekannten Sequenz des Gens OspA des Stammes AcaI von Bb unter Bezug auf den codierenden Strang des besagten Gens, ihre Halb-Hybridisierungstemperatur (Tm) bzw. ihre Größe wiedergibt, daß die Sequenzen in Richtung 5'→ 3' wiedergegeben werden, und daß die Sequenz des Primers BbGIII2 komplementär entgegengesetzt zur Sequenz auf dem codierenden Strang dargestellt wird.

9. Oligonukleotid gemäß Anspruch 1, enthaltend eine Sonde von für die Stämme von Bb der Gruppe II (besonders den Stamm B29) spezifischen Nukleinsäuren, entweder für das direkte Aufspüren oder für das indirekte Aufspüren über ein "Sandwich" genanntes System molekularer Hybridisierung, dadurch gekennzeichnet daß es das Oligomere:
| ***Referenz*** | ***Sequenz*** | ***Position*** | ***Größe*** | ***Temperatur*** |
|---|---|---|---|---|
| BbGII23 | ACTCTAGCTGCTGACGGCAAAAC | 508 - 530 | 23 | 70°C |
oder ein Oligomeres von invers komplementärer Sequenz enthält, mit der Maßgabe, daß A, T, C und G die zuvor genannten Bedeutungen haben, daß die obige Tabelle die Halb-Hybridisierungstemperatur (Tm), die Größe und Position der besagten Sequenz wiedergibt, die vom Ende 5' zum Ende 3' auf dem codierenden Strang des Gens OspA des Stamms B29 von Bb ausgerichtet ist, dessen Sequenz bekannt ist.

10. Oligonukleotid gemäß Anspruch 1, enthaltend eine Sonde von für die Stämme von Bb der Gruppe III (besonders den Stamm AcaI) spezifischen Nukleinsäuren, entweder für das direkte Aufspüren oder für das indirekte Aufspüren über ein "Sandwich" genanntes System molekularer Hybridisierung, dadurch gekennzeichnet, daß es das Oligomere:
| ***Referenz*** | ***Sequenz*** | ***Position*** | ***Größe*** | ***Temperatur*** |
|---|---|---|---|---|
| BbGIII28 | AGGAAAAGTAGCTAATGATAAAGTAACA | 503 - 530 | 28 | 70°C |
oder ein Oligomeres von invers komplementärer Sequenz enthält, mit der Maßgabe, daß A, T, C und G die zuvor genannten Bedeutungen haben, daß die obige Tabelle die Halb-Hybridisierungstemperatur (Tm), die Größe und Position der besagtenSequenz wiedergibt die vom Ende 5' zum Ende 3' auf dem codierenden Strang des Gens OspA des Stamms AcaI von Bb ausgerichtet ist, dessen Sequenz bekannt ist.

11. Oligonukleotid gemäß einem der Ansprüche 9 und 10, wenn es für das indirekte Aufspüren über ein "Sandwich" genanntes System molekularer Hybridisierung als für die betreffende Gruppe von Stämmen von Bb (bzw. II und III) spezifische Erfassungssonde eingesetzt wird in Kombination mit einer Anzeigesonde, welche ein Oligomeres Bb35bio oder Bb23bio oder sein Gegenstück enthält wie in Anspruch 3 definiert, chemisch modifiziert durch Einfügen einer oder mehrerer Gruppierungen am 5'- und/oder 3'-Ende, welche die Anzeige durch Einfärben oder Chemilumineszenz ermöglichen.

12. Oligonukleotid gemäß einem der Ansprüche 9 und 10, wenn es in Kombination mit einer allen Gruppen der Stämme von Bb gemeinsamen Erfassungssonde eingesetzt wird für das indirekte Aufspüren über ein "Sandwich" genanntes System molekularer Hybridisierung als für die betreffende Gruppe von Stämmen von Bb (bzw. II und III) spezifische Anzeigesonde, wobei diese Anzeigesonde chemisch modifiziert ist durch Einfügen einer oder mehrerer Gruppierungen am 5'- und/ oder 3'-Ende, welche die Anzeige durch Einfärben oder Chemilumineszenz ermöglichen.

13. Verfahren zum Aufspüren von Borrelia burgdorferi (Bb) bestehend aus der Herstellung eines molekularen Hybrids zwischen einer Erfassungssonde und der vorher denaturierten DNA von Bb und aus dem Einsatz einer Anzeigesonde zur Anzeige des Vorhandenseins des vorerwähnten molekularen Hybrids, dadurch gekennzeichnet, daß man eine Erfassungssonde wie in Anspruch 4 definiert und eine Anzeigesonde wie in Anspruch 5 definiert verwendet.

14. Verfahren zum Aufspüren von Borrelia burgdorferi (Bb) bestehend aus der Herstellung eines molekularen Hybrids zwischen einer Erfassungssonde und der vorher denaturierten DNA von Bb und
aus dem Einsatz einer Anzeigesonde zur Anzeige des Vorhandenseins des vorerwähnten molekularen Hybrids, dadurch gekennzeichnet, daß man eine für die Stämme von Bb einer der Gruppen I, II und III spezifische Erfassungssonde und eine Anzeigesonde wie in Anspruch 5 definiert verwendet.

15. Verfahren gemäß Anspruch 14, dadurch gekennzeichnet, daß die verwendete Erfassungssonde für die Stämme von Bb der Gruppe I (besonders den Stamm B31) spezifisch ist.

16. Verfahren gemäß Anspruch 14, dadurch gekennzeichnet, daß die verwendete Erfassungssonde für die Stämme von Bb der Gruppe II (besonders den Stamm B29) spezifisch und wie in Anspruch 9 definiert ist.

17. Verfahren gemäß Anspruch 14, dadurch gekennzeichnet, daß die verwendete Erfassungssonde für die Stämme von Bb der Gruppe III (besonders den Stamm AcaI) spezifisch und wie in Anspruch 10 definiert ist.

18. Verfahren zum Aufspüren von Borrelia burgdorferi (Bb), bestehend aus der Herstellung eines molekularen Hybrids zwischen einer Erfassungssonde und der vorher denaturierten DNA von Bb und aus dem Einsatz einer Anzeigesonde zur Anzeige des Vorhandenseins des vorerwähnten molekularen Hybrids, gekennzeichnet durch den Einsatz einer allen Stämmen von Bb gemeinsamen Erfassungssonde und einer für die Stämme von Bb einer der Gruppen I, II und III spezifischen Anzeigesonde.

19. Verfahren gemäß Anspruch 18, dadurch gekennzeichnet, daß die Meßsonde für die Stämme von Bb der Gruppe I (besonders den Stamm B31) spezifisch ist.

20. Verfahren gemäß Anspruch 18, dadurch gekennzeichnet, daß die Anzeigesonde für die Stämme von Bb der Gruppe II (besonders den Stamm B29) spezifisch und wie in Anspruch 9 definiert ist.

21. Verfahren gemäß Anspruch 18, dadurch gekennzeichnet, daß die Anzeigesonde für die Stämme von Bb der Gruppe III (besonders den Stamm AcaI) spezifisch und wie in Anspruch 10 definiert ist.

22. Verfahren zum Aufspüren von Borrelia burgdorferi (Bb) durch Genamplifikation der für diesen Mikroorganismus charakteristischen Fragmente denaturierter DNA, bestehend aus der Hybridisierung eines Paares Primer zur Ziel-DNA, der Synthese einer zur Ziel-DNA komplementären DNA und der Dissoziation der im Verlauf der beiden ersten Schritte gebildeten Hybride, gekennzeichnet durch den Einsatz eines Paares Primer, die geeignet sind für die Genamplifikation der DNA aller Gruppen bekannter Stamme von Bb, wobei dieses Paar Primer im Anspruch 2 definiert ist.

23. Verfahren gemäß Anspruch 22, gekennzeichnet durch den Einsatz eines Paares Primer, die für die Genamplifikation der Stämme von Bb der Gruppe I (besonders den Stamm B31) verwendet werden können, wobei dieses Paar Primer im Anspruch 6 definiert ist.

24. Verfahren gemäß Anspruch 22, gekennzeichnet durch den Einsatz eines Paares Primer, die für die Genamplifikation der Stämme von Bb der Gruppe II (besonders den Stamm B29) verwendet werden können, wobei dieses Paar Primer im Anspruch 7 definiert ist.

25. Verfahren gemäß Anspruch 22, gekennzeichnet durch den Einsatz eines Paares Primer, die für die Genamplifikation der Stamme von Bb der Gruppe III (besonders den Stamm AcaI) verwendet werden können, wobei dieses Paar Primer unter denen ausgewählt ist, die im Anspruch 8 definiert sind.

26. Verwendung eines oder mehrerer Oligonukleotide nach einem der Ansprüche 1 bis 14, für die Herstellung eines Mittels für die Diagnose von Borrelia burgdorferi (Bb) in biologischen Proben von Mensch oder Tier, besonders für die Epidemiologie, die Untersuchung von Wirtstieren, die Unterstützung bei der Therapie durch Bb hervorgerufener Infektionen, die Überprüfung der Wirksamkeit von Impfungen und der Zusammensetzung der Impfstoffe.
